(19) Europäisches Patentamt
European Patent Office
Office européen des brevets

(11) EP 2 764 028 B1

(12) **FASCICULE DE BREVET EUROPEEN**

(45) Date de publication et mention de la délivrance du brevet:
**28.04.2021 Bulletin 2021/17**

(21) Numéro de dépôt: **12769419.8**

(22) Date de dépôt: **03.10.2012**

(51) Int Cl.:
***C08B 37/00*** (2006.01) ***A61K 8/73*** (2006.01)

(86) Numéro de dépôt international:
**PCT/EP2012/069549**

(87) Numéro de publication internationale:
**WO 2013/050427 (11.04.2013 Gazette 2013/15)**

(54) **PROCÉDÉ DE PRÉPARATION DE GALACTOMANNANES CATIONIQUES**

VERFAHREN ZUR HERSTELLUNG VON KATIONISCHEN GALACTOMANNANEN

METHOD FOR PREPARING CATIONIC GALACTOMANNANS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **03.10.2011 FR 1158914**

(43) Date de publication de la demande:
**13.08.2014 Bulletin 2014/33**

(73) Titulaire: **RHODIA OPERATIONS**
**93300 Aubervilliers (FR)**

(72) Inventeurs:
- **LE-THIESSE, Jean-Claude**
  **F-42100 Saint Etienne (FR)**
- **LOMEL, Sébastien**
  **F-38540 Saint Just Chaleyssin (FR)**
- **GISBERT, Thierry**
  **F-69780 Mions (FR)**

(74) Mandataire: **Cardon, Flavie et al**
**Rhodia Operations**
**Intellectual Assets Management**
**52, rue de la Haie-Coq**
**93306 Aubervilliers Cedex (FR)**

(56) Documents cités:
**EP-A2- 0 234 014**
**WO-A1-2008/058769**
**DE-A1-102005 020 552**
**US-A1- 2009 197 829**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention a pour objet un nouveau procédé de préparation de galactomannanes cationiques, notamment de guars cationiques.
Les galactomannanes sont des polysaccharides composés principalement d'unités galactose et mannose, dans lesquels les unités mannose sont liés par une liaison glucosidique 1-4-β et les unités galactose sont reliées aux unités mannose par une liaison 1-6-a. Chaque cycle des unités galactose ou mannose (unités de sucre) porte trois groupes hydroxyles libres qui sont disponibles pour une réaction chimique.
Le galactomannane est une fibre végétale soluble et acalorique présente dans les graines et sert de réserve de sucre lors de la germination. Elle est abondante dans l'albumen de graines de légumineuses, telle que la *cyamopsis tetragonoloba* (gomme de guar), de la *Caesalpinia spinosa* (gomme tara) et de la *Ceratonia siliqua* (gomme de caroube).
Les galactomannanes naturels et modifiés sont essentiellement utilisés sous la forme de poudres (farines) dans de nombreux domaines, par exemple dans le domaine pétrolier, textile, alimentaire, pharmaceutique, cosmétique, dans l'industrie papetière, ou encore dans les explosifs ou dans le traitement de l'eau. Les galactomannanes naturels sont notamment utilisés depuis de nombreuses années dans la fabrication du papier pour améliorer la résistance du papier.
Afin d'améliorer les propriétés des galactomannanes, il est notamment possible de modifier les galactomannanes pour les rendre cationiques.
Ainsi, le brevet US 4 940 784 décrit un procédé de cationisation à sec de galactomannanes comprenant la réaction avec des alkylène époxydes en présence d'eau dans un milieu alcalin et de silice hydrophobe fine. Ce procédé permet de s'affranchir des étapes de séchage. Toutefois, l'utilisation de silice génère alors un produit dont les dispersions dans l'eau sont troubles. Enfin, ce procédé de l'état de la technique ne permet d'atteindre que des sélectivités faibles, à savoir inférieures à 50%, telles que mesurées pour le produit final après stockage et comme définies ci-après.
Le brevet US2009/197829 décrit quant à lui un procédé pour préparer un guar cationique, dans lequel on fait réagir les guars avec un composé ammonium quaternaire en présence d'une base.
La présente invention a pour but de fournir un procédé de préparation de galactomannanes cationiques amélioré, permettant d'obtenir une ou plusieurs des améliorations suivantes :

- une sélectivité élevée, notamment une sélectivité supérieure ou égale à 50%, notamment supérieure ou égale à 60%, par exemple supérieure ou égale à 70%, par exemple supérieure ou égale à 80% ; et/ou
- un taux d'humidité satisfaisant, notamment un taux d'humidité inférieur ou égal à 10%, par exemple inférieur ou égal à 7%, par exemple inférieur ou égal à 5% en sortie d'étape de séchage ; et/ou
- aboutissant à l'obtention d'un produit final sous forme d'une poudre dont la dispersion aqueuse est limpide ; et/ou
- de mise en œuvre plus économique et/ou plus écologique, notamment à l'échelle industrielle,

tout en conduisant à des galactomannanes cationiques présentant des propriétés comparables à ceux obtenus à partir des procédés de l'art antérieur, notamment en terme de modification de la viscosité et/ou de dépôt de silicones.
Ainsi, la présente invention concerne un procédé de préparation de galactomannane cationique, comprenant les étapes suivantes :

a) une étape d'imprégnation de galactomannane sous forme de splits par un agent alcalin ;
b) une étape d'imprégnation par un agent cationique du mélange formé à l'issue de l'étape a) ; et
c) une étape de séchage du mélange formé à l'issue de l'étape b).

Les étapes a) et b) sont effectuées dans des conditions adaptées pour obtenir de bonnes conditions d'imprégnation.
Dans le cadre de la présente invention, les conditions d'imprégnation sont telles que l'imprégnation est homogène, c'est-à-dire que chaque particule de galactomannane reçoit sensiblement la même quantité d'agent alcalin (étape a) et d'agent cationique (étape b).
Ces étapes d'imprégnation sont effectuées pendant un temps suffisant pour que l'agent alcalin et l'agent cationique se répartissent de la façon de la plus homogène dans le galactomannane.
Le procédé selon l'invention peut être indifféremment effectué en mode continu ou en batch.
Selon un mode de réalisation, le procédé de l'invention est effectué en continu.
Dans le cadre du procédé de l'invention, le galactomannane est sous forme de splits.
Le terme "splits de galactomannane" désigne une forme particulière des galactomannanes. Cette forme correspond à l'endosperme de la plante dont est issu le galactomannane. Elle consiste en des objets solides de galactomannanes présentant une taille de 3 à 4 mm.
Dans le cadre de la présente invention, l'imprégnation est encore plus efficace lorsque les particules de galactomannanes sont sous forme de splits. Dans ce cas, les agents alcalin et cationique sont absorbés plus lentement que pour les poudres et sont donc encore mieux répartis entre les particules. La répartition des agents alcalin et cationique est donc améliorée et encore plus homogène.

L'utilisation de galactomannanes sous forme de splits, permet donc une imprégnation particulièrement efficace et homogène.

Selon un mode de réalisation, les galactomannanes cationiques obtenus à l'issue du procédé de l'invention présentent un degré de substitution cationique $DS_{cat}$ allant de 0,1 à 0,3.

Au sens de la présente invention, on entend par « degré de substitution cationique $DS_{cat}$ » le nombre moyen de moles de groupes cationiques par mole d'unité de galactomannane. Ce degré de substitution cationique peut être mesuré par $^1$H-RMN (solvant : $D_2O$ ou DMSO).

Selon un mode de réalisation, le procédé de l'invention permet de façon avantageuse d'atteindre des sélectivités, mesurées après l'étape de séchage c), supérieures ou égales à 50%, notamment supérieures ou égales à 60%, par exemple supérieures ou égales à 70%, voire supérieures ou égales à 80%.

Au sens de la présente invention, on entend par « sélectivité » du procédé de cationisation, le rapport entre le nombre de groupes cationiques greffés sur le produit final et le nombre de groupes cationiques introduits dans le milieu réactionnel.

La sélectivité peut être déterminée en faisant le rapport entre le $DS_{cat}$ réel mesuré par $^1$H-RMN (solvant : $D_2O$ ou DMSO) à l'issue du procédé (sur un échantillon préalablement lavé) et le $DS_{cat}$ maximal théorique calculé en fonction des quantités molaires totales de réactifs introduites dans le milieu réactionnel.

Sauf indication contraire, la pression appliquée est la pression atmosphérique (1 bar).

L'expression "agent alcalin" désigne un agent basique notamment choisi dans le groupe constitué des silicates de métaux alcalins, des aluminates de métaux alcalins, des hydroxydes de métaux alcalins, des oxydes de métaux alcalins, des carbonates de métaux alcalins, des hydroxydes de métaux alcalino-terreux, des oxydes de métaux alcalino-terreux, et de leurs mélanges.

Selon un mode de réalisation particulier, l'agent alcalin utilisé pour l'étape a) est une solution aqueuse de soude ou de potasse, et préférentiellement de soude.

A l'issue de l'étape a), on obtient du galactomannane, sous forme de splits, imprégné d'agent alcalin.

L'étape b) consiste à ajouter un agent cationique au galactomannane imprégné d'agent alcalin.

Cette étape a pour but de greffer des groupements cationiques sur les galactomannanes. A l'issue de cette étape on obtient des galactomannanes cationiques, ou encore nommés ci-après galactomannanes greffés.

L'expression "agent cationique" désigne un composé portant au moins un groupe cationique. Dans ce qui suit, le terme "groupes cationiques" désigne des groupes chargés positivement ainsi que des groupes partiellement chargés positivement.

Le terme "groupes partiellement chargés positivement" désigne des groupes pouvant devenir chargés positivement en fonction du pH du milieu dans lequel ils se trouvent. Ces groupes peuvent également être nommés "groupes potentiellement cationiques".

Dans ce qui suit et ce qui précède, le terme "cationique" signifie également "au moins partiellement cationique".

L'agent cationique est également désigné "agent de greffage" ou "agent de cationisation". Le groupe cationique (ou les groupes cationiques) porté(s) par cet agent va se lier au galactomannane, via les groupements hydroxyles libres, pour former à l'issue du procédé de l'invention un galactomannane cationique, c'est-à-dire un galactomannane portant au moins un groupe cationique.

On peut ainsi représenter la réaction selon le schéma simplifié suivant :

galactomannane + agent cationique → galactomannane cationique imprégné

$G\ A\text{-}X^+ \rightarrow G\text{-}X^+$

Ainsi, dans le cadre de la présente invention, les termes "agent cationique" et "groupe cationique" englobent les composés ammoniums (avec une charge positive) mais également les composés amines primaires, secondaires et tertiaires ainsi que leurs précurseurs.

Les agents cationiques selon la présente invention peuvent être définis comme des composés qui, par réaction avec les groupes hydroxyles du galactomannane, peuvent conduire à la formation d'un galactomannane modifié (galactomannane cationique).

Selon la présente invention, on peut citer comme exemples d'agents cationiques les composés suivants :

- les époxydes cationiques, comme le chlorure de 2,3-époxypropyltriméthylammonium ou le bromure de 2,3-époxypropyltriméthylammonium ;
- les composés azotés cationiques comme le chlorure de 3-halogéno-2-hydroxypropyl triméthylammonium, par exemple le chlorure de 3-chloro-2-hydroxypropyl triméthylammonium ;

- les monomères éthylèniquement insaturés cationiques ou leurs précurseurs, comme le sel de chlorure du triméthylammoniumpropyl méthacrylamide, le sel de méthylsulfate de triméthylammoniumpropyl méthacrylamide, le chlorure de diallyl diméthyl ammonium, le chlorure de vinyl benzyl triméthylammonium, le diméthylaminopropyl méthacrylamide (amine tertiaire) ou leurs précurseurs, tels que le N-vinyl formamide, le N-vinylacétamide (dont les unités peuvent être hydrolysées après polymérisation ou greffage d'unités vinyl aminé).

Après l'étape b), les galactomannanes cationiques sont soumis à une étape de séchage.

Selon un mode de réalisation préféré, le galactomannane est choisi parmi les guars (gommes de guars) ou leurs dérivés. La présente invention concerne donc de préférence un procédé de préparation en continu de guar cationique, le guar étant sous forme de splits.

Selon un mode de réalisation, le procédé de l'invention comprend, après l'étape c), une étape de broyage du mélange séché obtenu à l'issue de l'étape c).

Cette étape de broyage peut notamment permettre de transformer les splits de galactomannane, notamment les splits de guar, en une poudre.

La poudre ainsi obtenue donne, une fois dispersée dans l'eau, une dispersion parfaitement limpide, contrairement par exemple aux poudres obtenues par les procédés de l'état de la technique décrit dans le brevet US 4 940 784 qui donnent des dispersions troubles. Ceci peut être avantageux lorsque des propriétés de transparence sont recherchées dans le produit final destiné à contenir le galactomannane cationique.

Les différentes étapes du procédé de l'invention vont maintenant être décrites plus en détail.

***Etape a) - imprégnation par l'agent alcalin***

La première étape du procédé consiste à introduire les splits de galactomannane, dans un récipient.

Selon un mode de réalisation, les splits de galactomannane, sont placés dans un récipient permettant de contrôler la température à laquelle ils sont maintenus, comme par exemple un mélangeur intensif équipé d'une double enveloppe dans laquelle circule un fluide caloporteur.

Par mélangeur intensif, on entend par exemple un mélangeur à socs de charrue ou un mélangeur à palettes, mono-axe ou bi-axe, ces outils pouvant être continus ou discontinus. On peut également utiliser des mélangeurs équipés de pales racleuses en fond de cuve tel qu'une turbosphère (ce type d'outil est discontinu). Sont également adaptés les mélangeurs à ressort qui sont des outils exclusivement continus. Ces exemples ne sont bien entendu pas limitatifs.

Les splits de galactomannanes, sont portés à une température T1 inférieure à 90°C, notamment inférieure à 80°C, de préférence inférieure à 70°C, et préférentiellement inférieure à 65°C, par exemple comprise entre 10°C et 65°C.

Selon un mode de réalisation, T1 est comprise de 55°C à 65°C, et de préférence égale à environ 60°C.

Les splits de galactomannanes sont mis en mouvement grâce à l'agitateur. La vitesse d'agitation dans le mélangeur est fixée de façon à avoir un renouvellement très fréquent des galactomannanes en contact avec les parois du mélangeur, au moins une fois par seconde, ceci dans le but de garantir un bon transfert de chaleur aux parois et d'avoir dans les étapes ultérieures une répartition homogène des réactifs liquides dans toute la masse de galactomannanes. La vitesse de rotation de l'agitateur permettant ce bon renouvellement à la paroi dépend de la technologie du mélangeur et de sa taille.

On additionne ensuite dans le même récipient une solution aqueuse de l'agent alcalin sur les galactomannanes maintenus à une température T1 telle que définie ci-dessus. De préférence, on utilise une solution aqueuse de soude.

Ainsi, selon un mode de réalisation particulier, une solution aqueuse de soude est introduite sur la masse de galactomannanes, sous forme de splits, en mouvement. Cette étape d'introduction peut être effectuée par coulée ou par pulvérisation.

Selon un mode de réalisation du procédé selon la présente invention, la solution aqueuse de l'agent alcalin est ajoutée à une température T2 inférieure à 90°C, notamment inférieure à 80°C, de préférence inférieure à 70°C, et préférentiellement inférieure à 65°C, par exemple comprise entre 10°C et 65°C.

Selon un mode de réalisation, T2 est comprise de 55°C à 65°C, et de préférence égale à environ 60°C.

Ainsi, avant l'étape d'addition, la solution aqueuse d'agent alcalin est préchauffée à une température T2 telle que définie ci-dessus.

Selon un mode de réalisation, le procédé de l'invention comprend une étape de préchauffage d'une solution de soude à environ 60°C avant introduction sur les galactomannanes, la vitesse d'introduction n'étant pas critique.

Selon un mode de réalisation du procédé de la présente invention, l'étape d'addition de l'agent alcalin est suivie d'une étape d'imprégnation des galactomannanes par la solution aqueuse de l'agent alcalin, qui consiste à se placer dans des conditions telles que l'imprégnation des galactomannanes par l'agent alcalin est satisfaisante et homogène.

La durée d'imprégnation t1 doit être suffisamment longue pour permettre à l'agent alcalin de diffuser de façon homogène dans la particule des splits de galactomannanes, avant de passer à l'étape suivante.

La durée d'imprégnation t1 est d'au moins 1 minute, par exemple d'au moins 5 minutes, sachant qu'il n'existe pas de durée maximale pour l'imprégnation. A titre illustratif, la durée d'imprégnation peut être par exemple comprise entre 1 minute et 120 minutes, notamment entre 5 minutes et 60 minutes.

Selon un mode de réalisation, notamment lorsque l'étape de séchage est effectuée très rapidement, par exemple sous la forme d'un séchage « flash » tel que défini ci-après, la durée d'imprégnation t1 peut être au moins égale à 15 minutes, par exemple comprise entre 15 minutes et 30 minutes, par exemple au moins égale à 20 minutes, par exemple comprise entre 20 minutes et 30 minutes.

Selon un autre mode de réalisation, notamment lorsque l'étape de séchage est effectuée dans des conditions permettant la poursuite de la réaction de greffage (conditions de séchage réactif tel que défini ci-après), la durée d'imprégnation t1 peut être au moins égale à 5 minutes, par exemple comprise entre 5 minutes et 15 minutes, par exemple comprise entre 5 minutes et 10 minutes.

Cette durée t1 est fonction de T4, la température des galactomannanes humidifiés, à savoir des galactomannanes mis en présence de l'agent alcalin, sous forme de splits. Cette température T4 dépend elle-même de T1, la température initiale des galactomannanes secs, à savoir les galactomannanes de départ avant toute introduction d'agent d'imprégnation, de T2, la température de la solution d'agent alcalin au moment de l'introduction et de T3, la température du fluide caloporteur circulant dans la double enveloppe du mélangeur.

Typiquement, lorsque T4 est de l'ordre de 25°C, t1 sera alors au minimum de 30 minutes et lorsque T4 est de l'ordre de 65°C, t1 est alors au minimum de 5 minutes, sachant qu'il n'existe pas de limite maximale pour t1.

Selon un mode de réalisation, lorsque le procédé est effectué en continu, on cherche à trouver les conditions (T1, T2, T3, t1) optimales d'un point de vue technico économique.

De préférence, le procédé est effectué dans des conditions telles que la température T4 des galactomannanes humides, sous forme de splits, est maintenue entre 55°C et 65°C, par exemple à environ 60°C dans le récipient. Préférentiellement, cette température ne doit pas dépasser 65°C.

De préférence, l'agitation est maintenue durant toute la durée t1 de l'étape d'imprégnation afin de garantir un renouvellement des particules de galactomannane au contact de la paroi du mélangeur et donc une température homogène dans la masse de particules.

Selon un mode de réalisation, l'étape a) est effectuée pendant une durée d'imprégnation t1 d'au moins 15 minutes, par exemple comprise entre 15 minutes et 40 minutes, par exemple d'au moins 20 minutes, par exemple comprise entre 20 et 30 minutes, à une température T4 comprise entre 55°C et 65°C.

***Etape b) - imprégnation par l'agent cationique***

Le procédé selon la présente invention comprend, après l'étape d'addition de l'agent alcalin et l'étape d'imprégnation, une étape d'addition d'une solution aqueuse de l'agent cationique.

L'agent cationique est introduit sur la masse de galactomannanes imprégnés d'agent alcalin maintenue en mouvement dans le récipient (de préférence mélangeur intensif) tel que défini ci-dessus. Cette étape d'introduction peut être effectuée par coulée ou par pulvérisation.

L'agent cationique peut notamment être choisi parmi les alkylène époxydes, et plus particulièrement parmi les composés suivants :

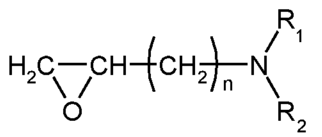

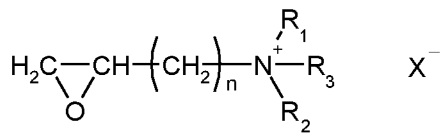

n représentant un nombre entier de 1 à 3,

$R_1$, $R_2$ et $R_3$ représentant indépendamment les uns des autres un groupe alkyle comprenant de 1 à 4 atomes de carbone, ou $R_1$ pouvant représenter un groupe benzyle,

$X^-$ représentant $Cl^-$, $Br^-$ ou $AcO^-$.

A titre d'agent cationique, on peut notamment citer les composés suivants :

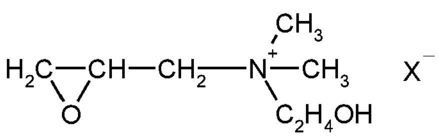

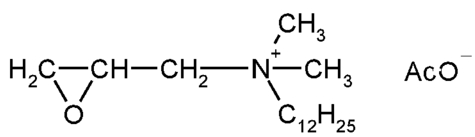

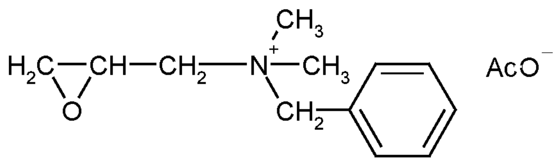

$X^-$ étant tel que défini ci-dessus.

On peut également utiliser les agents cationiques de formule générale suivante :

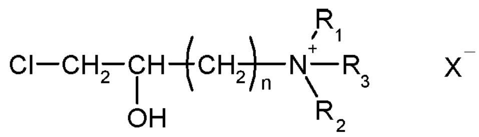

n, $R_1$, $R_2$, $R_3$ et $X^-$ étant tels que définis ci-dessus.

De préférence, l'agent cationique est le chlorure de 3-chloro-2-hydroxypropyltriméthylammonium (Quab®188).

On peut également citer le chlorure de 2,3-époxypropyltriméthylammonium (Quab® 151).

Selon un mode de réalisation du procédé de l'invention, une solution aqueuse de Quab® 188 est introduite sur la masse de galactomannanes, sous forme de splits, en mouvement, par exemple par coulée

Selon un mode de réalisation, la concentration massique de la solution d'agent cationique est de 5% à 95%, et de préférence égale à 65%, en agent cationique. On peut par exemple utiliser une solution aqueuse comprenant 65% en poids de Quab® 188, telle que commercialisée par la société Fluka.

Avant d'être introduite sur la masse de galactomannane en mouvement, ladite solution d'agent cationique est préchauffée à une température T5 inférieure à 90°C, notamment inférieure à 80°C, de préférence inférieure à 70°C, et préférentiellement inférieure à 65°C, par exemple comprise entre 10°C et 65°C.

Selon un mode de réalisation, T5 est comprise de 55°C à 65°C.

De préférence, la solution d'agent cationique, de préférence de Quab® 188, est préchauffée à environ 60°C avant introduction sur les galactomannanes, la vitesse d'introduction n'étant pas critique.

Selon un mode de réalisation du procédé de la présente invention, l'étape d'addition de l'agent cationique est suivie d'une étape d'imprégnation des galactomannanes issus de l'étape a) par la solution aqueuse de l'agent cationique.

La durée d'imprégnation t2 doit être suffisamment longue pour permettre à l'agent cationique de diffuser jusque dans les particules des splits de galactomannanes, avant de passer à l'étape de séchage.La durée d'imprégnation t2 est d'au moins 1 minute, par exemple d'au moins 5 minutes, sachant qu'il n'existe pas de durée maximale pour l'imprégnation. A titre illustratif, la durée d'imprégnation peut être par exemple comprise entre 1 minute et 120 minutes, notamment entre 5 minutes et 60 minutes.

Selon un mode de réalisation, notamment lorsque l'étape de séchage est effectuée très rapidement, par exemple sous la forme d'un séchage « flash » tel que défini ci-après, la durée d'imprégnation t2 peut être au moins égale à 15 minutes, par exemple comprise entre 15 minutes et 30 minutes, par exemple au moins égale à 20 minutes, par exemple comprise entre 20 minutes et 30 minutes.

Selon un autre mode de réalisation, notamment lorsque l'étape de séchage est effectuée dans des conditions permettant la poursuite de la réaction de greffage (conditions de séchage réactif tel que défini ci-après), la durée d'imprégnation t2 peut être au moins égale à 5 minutes, par exemple comprise entre 5 minutes et 30 minutes, par exemple comprise entre 5 minutes et 15 minutes.

Comme précédemment pour l'agent alcalin, cette durée t2 est fonction de T6, la température des galactomannanes humidifiés, à savoir des galactomannanes mis en présence de l'agent cationique, sous forme de splits. Cette température T6 dépend elle-même de T4, la température des galactomannanes à l'issue de l'étape d'imprégnation par l'agent alcalin, de T5, la température de la solution d'agent cationique au moment de l'introduction et de T3, la température du fluide caloporteur circulant dans la double enveloppe du mélangeur.

Typiquement, lorsque T6 est de l'ordre de 25°C, t2 sera alors au minimum de 30 minutes et lorsque T6 est de l'ordre de 65°C, t2 est alors au minimum de 5 minutes, sachant qu'il n'existe pas de limite maximale pour t2.

Selon un mode de réalisation, l'étape b) est effectuée pendant une durée d'imprégnation t2 d'au moins 15 minutes, par exemple comprise entre 15 minutes et 40 minutes, par exemple d'au moins 20 minutes, par exemple comprise entre 20 et 30 minutes, à une température T6 comprise entre 55°C et 65°C.

Selon un mode de réalisation du procédé de l'invention, l'agent alcalin est utilisé en excès par rapport à l'agent cationique.

Selon un mode de réalisation du procédé, le rapport entre le nombre de moles de l'agent alcalin et le nombre

de moles de l'agent cationique est compris de 1,5 à 2,5, et de préférence égale à 1,7.

Par exemple, avec un ratio égal à 2,1 on atteint une sélectivité de 85% après séchage tandis que lorsque ce ratio est inférieur à 1,5, par exemple égal à 1,3, on diminue la sélectivité à 70%.

Ainsi, selon un mode de réalisation préféré, le procédé de l'invention est effectué avec un ratio molaire soude / Quab® 188 égal à 1,7.

Dans le cadre du procédé de la présente invention, la quantité des différents réactifs varie selon le DS visé (choisi selon les applications visées). Il est donc des compétences de l'homme du métier, de choisir les quantités à utiliser compte tenu des DS visés et des sélectivités obtenues.

Selon le procédé de la présente invention, l'ordre des réactifs est important. De préférence, la totalité de l'agent alcalin doit être ajoutée avant l'agent cationique.

En effet, il a été constaté que si l'agent cationique est introduit en premier, c'est-à-dire avant l'agent alcalin, celui-ci diffuse plus lentement jusqu'au cœur des des splits de galactomannanes. Après ajout de la soude, les galactomannanes restent collants et difficiles à sécher. De plus, la sélectivité est médiocre (de l'ordre de 48%).

De préférence, dans le cadre du procédé de l'invention, la température des galactomannanes humides pendant l'imprégnation, ne doit pas dépasser 65°C.

En présence d'eau, d'agent alcalin et d'agent cationique, les réactions de dégradation de l'agent cationique, notamment du Quab, s'accélèrent fortement à partir de 65°C pour former des sous-produits, la sélectivité de la réaction s'en trouvant dégradée.

***Etape c) - séchage***

A l'issue des étapes a) et b), le milieu réactionnel comprenant les galactomannanes cationisés humides est séché. Par exemple, ledit mélange peut être séché in situ dans le mélangeur ou transféré vers un séchoir.

Selon l'invention, cette étape de séchage peut être effectuée très rapidement ou dans des conditions contrôlées permettant la poursuite du greffage. La durée de séchage est donc comprise de 1 seconde à 180 minutes.

La température de séchage peut varier de 60°C à 350°C, et ce en fonction du mode de séchage et de la durée de séchage.

Lorsque le séchage est effectué très rapidement, à savoir de l'ordre de la seconde, la température de séchage est élevée, notamment de l'ordre de 300°C, voire jusque 350°C. Dans ce cas, l'étape de séchage est une étape de séchage "flash".

Selon un mode de réalisation, lorsque le séchage est de type séchage "flash", les étapes de séchage et de broyage peuvent être simultanées. On parle alors de séchage-broyage flash.

Selon un autre mode de réalisation, l'étape de séchage est effectuée dans des conditions contrôlées, par exemple par circulation d'air, dans des conditions telles que la réaction de greffage peut se poursuivre. On parle alors de séchage réactif.

Au cours de cette étape de séchage, la réaction de greffage se poursuit, ce qui permet d'obtenir des galactomannanes cationiques avec un taux de greffage satisfaisant et une sélectivité encore plus élevée.

Dans le cadre de ce mode de réalisation, la température T7 de l'air de séchage peut être supérieure ou égale à 60°C, notamment comprise entre 60°C et 150°C, de préférence entre 60°C et 100°C.

Selon un mode de réalisation préféré, la température de l'air de séchage est fixée à environ 80°C.

La durée de séchage est adaptée pour que l'humidité finale des galactomannanes, splits, greffés soit inférieure à 5%.

Ainsi, selon un mode de réalisation, la durée de séchage t4 de l'étape c) de séchage réactif du procédé de l'invention est supérieure ou égale à 5 minutes, par exemple supérieure ou égale à 10 minutes.

Selon un mode de réalisation préféré, cette durée de séchage est comprise de 10 minutes à 180 minutes, et est de préférence supérieure ou égale à environ 15 minutes.

Ainsi, un séchage selon ce mode de réalisation laisse le temps à la réaction de greffage de se poursuivre jusqu'à son terme : le gain de sélectivité pendant le séchage est donc plus élevé.

Il est préférable de contrôler la température des produits obtenus (galactomannanes) en fin de séchage, et ce à une température comprise de 60°C à 80°C. Pour ce faire, il est donc avantageux d'effectuer le séchage à des températures contrôlées inférieures à 150°C.

Si la température des galactomannanes est inférieure à 60°C, le potentiel de greffage pendant le séchage n'est pas exploité totalement et la sélectivité finale est abaissée (à environ 70 %).

Si la température des galactomannanes dépasse 80°C en fin de séchage, on peut observer un début de dégradation qui se manifeste par un brunissement du produit.

Selon un mode de réalisation, les galactomannanes cationiques obtenus à l'issue de l'étape de séchage du procédé de l'invention présentent un taux d'humidité inférieur à 5%.

Au-dessus de cette valeur de 5% de taux d'humidité, les galactomannanes obtenus restent plastiques et

difficiles à broyer.

Ce taux d'humidité est mesuré par perte de poids à 80°C à l'aide d'une thermobalance halogène.

Ainsi, en adoptant une température de l'air de séchage égale à 80°C afin de garantir que les galactomannanes secs ne dépassent pas cette température, la durée de séchage réactif pour atteindre moins de 5% d'humidité résiduelle est de l'ordre de 15 minutes.

Le choix de la technologie de séchage est large et dépendra des conditions opératoires retenues pour l'exploitation. De préférence, on utilise un séchoir convectif agité en raison de la grande homogénéité de séchage obtenue. On utilise par exemple un lit fluidisé, éventuellement vibré, ou un tambour rotatif. Bien que le choix technologique ne soit pas critique pour la qualité du produit final (un séchage parfaitement satisfaisant peut être obtenu en 3 heures dans une étuve à 60°C), un mode de réalisation particulièrement préféré consiste à effectuer un séchage en lit fluidisé agité.

***Etape d) - broyage***

Les galactomannanes étant sous forme de splits, ceux-ci doivent être transformés sous forme de poudre. Dans ce cas, l'étape c) est suivie d'une étape d) de broyage afin d'obtenir une poudre de galactomannane cationique comprenant des particules de taille souhaitée.

Le procédé de l'invention présente plusieurs avantages qui contribuent à la réduction du prix de revient du produit fini et/ou à de meilleures performances environnementales.

Tout d'abord, le choix spécifique de certaines conditions d'imprégnations particulières permet d'améliorer significativement la sélectivité de la réaction de greffage (ou cationisation).

En outre, selon un mode préféré de réalisation, la réaction de greffage (ou cationisation) se poursuit pendant le séchage, ce qui permet d'atteindre une sélectivité finale encore plus importante, et notamment supérieure à 50%, voire supérieure à 80%. Ainsi, ce procédé permet, pour un taux de greffage visé, de réduire la quantité d'agent cationique engagée dans le procédé.

Le fait de poursuivre la réaction de greffage de façon contrôlée pendant le séchage permet d'atteindre des sélectivités de 80%, voire supérieures. Or, le fait de laisser évoluer le produit au stockage sans étape de séchage, conformément aux procédés de l'état de la technique, conduit à des niveaux de sélectivité moindre et à des taux d'impureté importants.

Le procédé selon l'invention présente également l'avantage de ne pas nécessiter d'étape de lavage, ce qui supprime l'investissement des équipements nécessaires à cette opération, réduit les temps de procédé et supprime l'immense majorité des effluents aqueux. Par ailleurs, l'absence d'étape de lavage conduit à un produit contenant moins d'humidité, d'où un gain énergétique au séchage.

Par ailleurs, le temps de séjour dans le mélangeur est considérablement réduit, ce qui limite la taille des équipements nécessaires pour une productivité donnée et permet, si on le souhaite, de développer un procédé continu pour les étapes d'imprégnation par l'agent alcalin puis par l'agent cationique.

Selon un mode de réalisation, le procédé de l'invention ne comprend pas l'utilisation de silice dans les étapes a), b) et c).

Par ailleurs, le procédé de l'invention ne comprend pas d'étape de lavage.

Selon un mode de réalisation, le procédé de l'invention peut comprendre des étapes supplémentaires, et en particulier une étape de dépolymérisation et/ou de réticulation des galactomannanes.

Par exemple, le procédé de l'invention peut comprendre une étape de dépolymérisation des galactomannanes. Cette étape est effectuée avant l'étape b).

Cette étape de dépolymérisation est effectuée par l'utilisation d'un agent de dépolymérisation des galactomannanes. Typiquement, à titre d'agent de dépolymérisation, on peut notamment utiliser les agents oxydants, tels que notamment le peroxyde d'hydrogène, acide nitrique, et leurs mélanges, ou les acides, tels que notamment l'acide lactique, l'acide tartrique, l'acide citrique, l'acide phosphorique, l'acide sulfurique et leurs mélanges.

Par exemple, l'agent de dépolymérisation peut être introduit sur les galactomannanes, sur les splits, avant, après ou en même temps que l'agent alcalin.

Le procédé de l'invention peut également comprendre une étape de réticulation des galactomannanes. Cette étape est effectuée avant et/ou après les étapes a) et b).

Cette étape de réticulation peut être effectuée par l'utilisation d'un agent de réticulation des galactomannanes.

A titre d'agent de réticulation, on peut par exemple utiliser un composé choisi parmi le formaldéhyde, le glyoxal, les halohydrines telles que l'épichlorhydrine ou répibromhydrine, l'oxychlorure de phosphore, les polyphosphates, les diisocyanates, la biséthylène urée, les polyacides tel que l'acide adipique, l'acide citrique, l'acroléïne et similaires. La réticulation chimique peut également être obtenue par l'action d'un complexant métallique tel que par exemple du Zirconium (IV).

La réticulation chimique peut également être obtenue sous l'effet d'un rayonnement ionisant.

Les exemples ci-après sont donnés à titre illustratif mais ne sont en aucun cas limitatifs.

## EXEMPLES

### Exemple 1

200 g de splits (Hindustan Gum & Chemicals), stockés à 60°C, sont introduits dans un mélangeur de laboratoire (Pro-C-epT, Mi-Pro 1900) équipé d'une cuve d'un volume de 2 litres. L'agitation est assurée par une tripale en fond de cuve et la vitesse de rotation est fixée à 100 tours par minute.
La cuve de ce mélangeur est munie d'une double enveloppe dans laquelle circule de l'eau à 65°C.
Une solution aqueuse de soude a été préalablement préparée par dissolution de 18,6 g de pastilles de soude (Normapur, VWR) dans 100 g d'eau. Cette solution, préchauffée à 60°C, est coulée en 30 secondes sur les splits en mouvement.
Après 5 minutes de mélange avec la solution de soude, 80,7 g d'une solution aqueuse à 65% en poids de Quab® 188 (Fluka) est coulée en 30 secondes sur les splits en mouvement. Cette solution de Quab® avait également été préchauffée à 60°C avant introduction dans le mélangeur.
Après 5 minutes de mélange avec la solution de Quab®, le mélangeur est vidangé. Une aliquote d'une dizaine de grammes de splits est immédiatement immergée dans 200 ml de méthanol pour stopper la réaction et pouvoir contrôler le degré de substitution cationique atteint en sortie de mélangeur.
Le reste des splits est étalé en couche mince sur un plateau métallique introduit pendant 3 heures dans une étuve à 60°C. A l'issue de ces 3 heures, l'humidité résiduelle des splits est contrôlée par perte de poids à 80°C à l'aide d'une thermobalance halogène. L'humidité mesurée est de 5%.
Les splits secs sont finalement broyés avec un broyeur à marteaux équipé d'une grille de 500 $\mu$m.
Les degrés de substitution ($DS_{cat}$) sont mesurés par RMN. On obtient :

- en sortie de mélangeur, $DS_{cat}$ = 0,10 soit une sélectivité de 40%
- en sortie de séchage, $DS_{cat}$ = 0,22 soit une sélectivité de 88%

### Exemple 2

Les conditions d'imprégnation de l'exemple 1 sont reprises à 1 différence près : les splits sont à 25°C au moment de leur introduction dans le mélangeur.
Après imprégnation par la solution de soude puis la solution de Quab 188, une aliquote d'une dizaine de grammes de splits est immédiatement immergée dans 200 ml de méthanol pour stopper la réaction et pouvoir contrôler le degré de substitution cationique atteint en sortie de mélangeur.
Le degré de substitution ($DS_{cat}$) mesuré par RMN est :

- en sortie de mélangeur, $DS_{cat}$ = 0,08 soit une sélectivité de 32%

Le reste des splits est subdivisé en 2 parts sensiblement égales.
La première partie est séchée en lit fluidisé pendant 10 minutes avec de l'air à 80°C. L'humidité résiduelle mesurée est de 5%. Les splits secs sont broyés avec un broyeur à marteaux équipé d'une grille de 500 $\mu$m.
La seconde partie est séchée et broyée simultanément. Pour cela, le broyeur à marteaux est traversé par un courant d'air à 270°C pendant toute la durée du broyage. Le temps de séjour du solide dans le broyeur est de 30 à 45 secondes. L'humidité résiduelle mesurée à l'issue de ce séchage flash est de 7.6%.
Les degrés de substitution ($DS_{cat}$) mesurés par RMN sont :

- après séchage en lit fluidisé, $DS_{cat}$ = 0,19 soit une sélectivité de 76%
- après séchage flash, $DS_{cat}$ = 0,12 soit une sélectivité de 48%

Cet exemple montre que des résultats satisfaisants peuvent également être obtenus lorsque les splits ne sont pas préchauffés.
Par ailleurs, cet exemple démontre qu'une étape de séchage réactif peut être effectuée en lit fluidisé, technologie aisément industrialisable.
Enfin, cet exemple illustre la différence de gain de sélectivité obtenu entre un séchage dans des conditions contrôlées et un séchage flash.

**Exemple 3**

Les conditions de l'exemple 2 sont reprises à 1 différence près :

- le séchage est réalisé en lit fluidisé pendant 30 minutes avec de l'air à 50°C.

L'humidité résiduelle mesurée est de 5%. Les degrés de substitution ($DS_{cat}$) mesurés par RMN sont :

- en sortie de mélangeur, $DS_{cat}$ = 0,07 soit une sélectivité de 28%
- en sortie de séchage, $DS_{cat}$ = 0,14 soit une sélectivité de 56%

Cet exemple démontre donc que la sélectivité est diminuée lorsque la température de séchage est insuffisamment élevée, c'est-à-dire inférieure à 60°C.

**Exemple 4**

Les conditions de l'exemple 2 sont reprises à 1 différence près :

- le temps de mélange avec la soude n'est que de 3 minutes avant introduction du Quab®

L'humidité résiduelle mesurée est de 5%. Les degrés de substitution ($DS_{cat}$) mesurés par RMN sont :

- en sortie de mélangeur, $DS_{cat}$ = 0,07 soit une sélectivité de 28%
- en sortie de séchage, $DS_{cat}$ = 0,14 soit une sélectivité de 56%

Cet exemple démontre donc que la sélectivité est diminuée lorsque le temps de mélange avec la soude est trop court, en l'espèce inférieur à 5 minutes.

**Exemple 5**

Les quantités de réactifs sont identiques à celles de l'exemple 1.
La température initiale des splits et des solutions de réactif est de 22 - 23°C ; la température du fluide caloporteur dans la double enveloppe du mélangeur est de 30°C.
Les durées d'imprégnation après ajout de la soude puis après ajout du Quab 188 sont toutes les deux de 30 minutes.
Le séchage est réalisé en lit fluidisé pendant 20 minutes avec de l'air à 80°C ; l'humidité résiduelle en fin de séchage est de 3,8%.
Les degrés de substitution ($DS_{cat}$) mesurés par RMN sont :

- en sortie de mélangeur, $DS_{cat}$ = 0,09 soit une sélectivité de 36%
- en sortie de séchage, $DS_{cat}$ = 0,22 soit une sélectivité de 88%

Cet exemple démontre que de très bons résultats en terme de sélectivité peuvent être obtenus à l'issue du séchage réactif si on adapte les temps d'imprégnation en fonction des températures de travail.

**Exemple 6**

Les quantités de réactifs sont identiques à celles de l'exemple 1.
La température initiale des splits est de 22 - 23°C. Les solutions de réactif (soude et Quab 188) sont préchauffées à 60°C avant introduction dans le mélangeur. La température du fluide caloporteur dans la double enveloppe du mélangeur est de 65°C.
Les durées d'imprégnation après ajout de la soude puis après ajout du Quab 188 sont toutes les deux de 30 minutes.
Un séchage flash est réalisé simultanément à l'étape de broyage. Pour cela, le broyeur à marteaux est traversé par un courant d'air à 270°C pendant toute la durée du broyage. Le temps de séjour du solide dans le broyeur est de 30 à 45 secondes. L'humidité résiduelle mesurée à l'issue de ce séchage flash est de 6.8%.

Les degrés de substitution ($DS_{cat}$) mesurés par RMN sont :

- en sortie de mélangeur, $DS_{cat}$ = 0,17 soit une sélectivité de 68%
- en sortie de séchage flash, $DS_{cat}$ = 0,19 soit une sélectivité de 76%

Cet exemple démontre que de bons résultats en terme de sélectivité peuvent être obtenus directement en sortie de mélangeur si on augmente les temps d'imprégnation et les températures de travail. Un séchage flash conduit alors à une sélectivité finale très satisfaisante.

## Exemple 7

Les références des réactifs utilisés sont ceux indiqués dans l'exemple 1.

200 g de splits, stockés à 22°C, sont introduits dans le mélangeur de laboratoire. La température du fluide caloporteur dans la double enveloppe du mélangeur est de 65°C.

Une solution aqueuse de soude a été préalablement préparée par dissolution de 14,9 g de pastilles de soude dans 80 g d'eau soit 20% de moins que dans les exemples précédents. Dans cette solution ont ensuite été dissous 0,40 g de borax décahydrate, agent de réticulation des galactomannanes. Cette solution, préchauffée à 60°C, est coulée en 30 secondes sur les splits en mouvement.

Immédiatement après la solution de soude, 6,7 g d'une solution aqueuse à 30% de peroxyde d'hydrogène est coulée sur les splits en mouvement. Le peroxyde d'hydrogène est un agent de dépolymérisation des galactomannanes. La température de cette solution aqueuse est de 22°C.

Après 30 minutes de mélange avec ces 2 solutions, 64,6 g d'une solution aqueuse à 65% en poids de Quab® 188 est coulée en 30 secondes sur les splits en mouvement. Comme pour la soude, la quantité de Quab® est donc réduite de 20% par rapport aux exemples précédents. Cette solution de Quab® avait également été préchauffée à 60°C avant introduction dans le mélangeur.

Après 30 minutes de mélange avec la solution de Quab®, 36 g d'une solution aqueuse à 10% d'acide chlorhydrique est coulée en 20 secondes sur les splits en mouvement. Cette ultime étape de la synthèse a pour but de neutraliser partiellement la soude en excès et de renforcer la réticulation par le borax après l'étape de cationisation.

Après 20 minutes de mélange avec la solution d'acide chlorhydrique, le mélangeur est vidangé. Une aliquote d'une dizaine de grammes de splits est immédiatement immergée dans 200 ml de méthanol pour stopper la réaction et pouvoir contrôler le degré de substitution cationique atteint en sortie de mélangeur.

Le reste des splits est séché et broyé simultanément. Pour cela, le broyeur à marteaux est traversé par un courant d'air à 270°C pendant toute la durée du broyage. Le temps de séjour du solide dans le broyeur est de 30 à 45 secondes. L'humidité résiduelle mesurée à l'issue de ce séchage flash est de 6.8 %.

Les degrés de substitution ($DS_{cat}$) mesurés par RMN sont :

- après séchage en lit fluidisé, $DS_{cat}$ = 0,10 soit une sélectivité de 50%
- après séchage flash, $DS_{cat}$ = 0,12 soit une sélectivité de 55%

Cet exemple illustre le cas où les étapes de dépolymérisation et de réticulation sont mises en œuvre.

## Exemple 8

Des compositions de shampooing contenant des galactomannanes cationiques selon l'invention ont été préparées afin d'évaluer les performances de ces galactomannanes cationiques en termes de dépôt de silicone.

Les galactomannanes cationiques préparés dans les exemples 1, 3 et 7 ont été incorporés dans une composition de shampoing, décrite dans le tableau ci-après. La quantité de chacun des composés est exprimée en pourcentage massique de la formulation totale en considérant la part active du composé.

| Composés | Nom commercial | Fournisseur | % massique en actif |
|---|---|---|---|
| Laureth sulfate de sodium | Rhodapex® ES-2K | Rhodia | 14 |
| Cocamidopropyl bétaïne | Mirataine® BET C-30 | Rhodia | 2 |
| Galactomannane selon l'invention | - | - | 0,3 |
| Emulsion de diméthicone | Mirasil® DM 500 000 | Bluestar Silicones | 1 |

(suite)

| Composés | Nom commercial | Fournisseur | % massique en actif |
|---|---|---|---|
| Chlorure de sodium | - | - | 1,8 |
| Acide citrique (pH 6,0-6,5) | - | - | Quantité suffisante |
| Conservateur | - | - | Quantité suffisante |
| Eau | - | - | Complément jusqu'à 100 |

Pour comparaison, des compositions de shampooing identiques ont été préparées en remplaçant le galactomannane cationique par des guars cationiques différents de ceux de l'invention, à savoir du Jaguar C17®, du Jaguar C14S® ou du Jaguar C500®.

L'efficacité de dépôt de silicone des shampooings ainsi préparés a été mesurée à l'aide d'une tresse de cheveux calibrée de référence Virgin Médium Brown Caucasian Hair fournie par la société IHIP (International Hair Importers & Products Inc.). La tresse de cheveux a une masse de 4,5 g et une longueur de 20 cm, l'une de ses extrémités comportant un clip de fixation.

La méthode de mesure comporte quatre étapes: le prétraitement des tresses de cheveux avec une solution de laureth sulfate de sodium (SLES) à 10%, le traitement des tresses de cheveux avec le shampooing à évaluer comprenant du diméthicone, l'extraction du diméthicone à l'aide de tétrahydrofurane (THF) et le dosage du diméthicone extrait par chromatographie sur gel perméable (GPC). Le protocole utilisé pour chacune de ces étapes est décrit de façon détaillée ci-après.

1. Prétraitement des tresses de cheveux

Les tresses de cheveux ont été prétraitées avec une solution de SLES à 10%, puis elles ont été rincées à l'eau avant l'étape suivante de traitement par le shampooing contenant du diméthicone.

Le protocole de prétraitement était le suivant : chaque tresse a été soumise à un flux d'eau contrôlé (150 ml/min à 38°C) pendant 1 minute, puis 3 ml de la solution de SLES à 10% ont été appliqués le long de la tresse. Enfin, la tresse a été rincée à l'eau pendant 1 minute.

2. Traitement des cheveux

Environ 450 mg de shampooing ont été pesés et la masse exacte a été notée précisément. La tresse de cheveux a été enroulée autour du doigt et le shampooing a été appliqué dessus. Puis, on a massé le shampooing sur la tresse pendant 45 secondes et on a veillé à ce que le produit soit appliqué de façon égale sur toute la tresse. Cette dernière a ensuite été rincée à l'eau pendant 30 secondes. L'excès d'eau a été retiré de la tresse en passant l'index et le majeur à travers la tresse et on a laissé la tresse sécher à l'air ambiant et s'équilibrer pendant une nuit dans une pièce climatisée (21°C, 50% d'humidité relative).

3. Extraction du diméthicone

Pour chacune des tresses, des bouteilles en polyéthylène de 250 ml ont été tarées. Chaque tresse a été introduite dans une bouteille tout en maintenant le clip de fixation à l'extérieur de la bouteille. Chaque tresse a ensuite été coupée juste en dessous du clip et la quantité de cheveux introduite dans chaque bouteille a été notée. Puis, environ 100 ml de THF ont été introduits dans chaque bouteille, avant que celle-ci soit refermée. Toutes les bouteilles ont été placées sur une plaque d'agitateur et laissées à agiter pendant 24 heures à 200 tours par minute. Sous une hotte, la solution d'extraction de THF a été transférée dans une capsule d'évaporation de 150 ml et laissée à évaporer (taux de ventilation maximal) pendant 24 heures sous la hotte. Après évaporation, la capsule d'évaporation ne contenait plus que le diméthicone extrait, déposé sur les parois.

4. Dosage du diméthicone extrait

La capsule d'évaporation a été tarée avec un verre de montre la recouvrant. Ensuite, sous la hotte, environ 4 ml de THF ont été introduits dans la capsule d'évaporation. A l'aide d'une spatule, le diméthicone déposé sur les parois de la capsule d'évaporation a été dissout à nouveau. Après dissolution complète, la capsule d'évaporation recouverte du verre de montre a été pesée et la quantité de THF introduite notée. A l'aide d'une seringue, la solution de diméthicone a été transférée dans un tube de 2 ml qu'on a ensuite refermé. La concentration de diméthicone a été dosée dans le

tube par GPC.

La quantité Q de diméthicone déposée sur les cheveux, exprimée en ppm (μg de diméthicone par g de cheveux), a été déterminée par la relation suivante :

$$Q = \frac{C_{\text{dim}\,éthicone} \times m_{THF}}{m_{cheveux}}$$

où $C_{diméthicone}$ est la concentration de diméthicone dans le tube de GPC, exprimée en ppm (μg de diméthicone par g de THF),
$m_{THF}$ la masse de THF, exprimée en g, utilisée pour solubiliser le diméthicone dans la capsule d'évaporation, et
$m_{cheveux}$, la masse de cheveux, exprimée en g, introduite dans la bouteille en polyéthylène.

Par ailleurs, le rendement de dépôt R a été déterminé par la relation suivante :

$$R\ (\%) = \frac{C_{\text{dim}\,éthicone} \times m_{THF}}{m_{shampooing} \times \phi}$$

où $m_{shampooing}$ est la masse de shampooing, exprimée en μg, utilisée pour traiter la tresse de cheveux, et
Φ la concentration de diméthicone dans le shampooing.

Deux tresses au minimum ont été utilisées pour chacune des compositions afin de calculer la quantité moyenne de diméthicone déposée sur les cheveux et le rendement de dépôt moyen.

Les résultats obtenus au test de dépôt de silicone pour chacun des exemples testés sont indiqués dans les tableaux ci-après.

La performance de l'Exemple 1 a été comparée à celle du Jaguar C17® :

| Guar utilisé | DS | Rendement (%) |
|---|---|---|
| Jaguar C17® | 0,20 | 57 |
| Exemple 1 | 0,22 | 59 |

La performance de l'Exemple 3 a été comparée à celle du Jaguar C14S® :

| Guar utilisé | DS | Rendement (%) |
|---|---|---|
| Jaguar C14S® | 0,15 | 48 |
| Exemple 3 | 0,14 | 51 |

La performance de l'Exemple 7 a été comparée à celle du Jaguar C500® :

| Guar utilisé | DS | Rendement (%) |
|---|---|---|
| Jaguar C500® | 0,10 | 26 |
| Exemple 7 | 0,12 | 30 |

Ces résultats démontrent que les rendements de dépôt de silicone obtenus avec les shampooings contenant des galactomannanes cationiques selon l'invention sont comparables à ceux obtenus avec des guars cationiques conventionnels, voire plus élevés.

Le procédé de l'invention présente ainsi l'avantage de réduire les temps de procédé et les coûts, tout en permettant de préparer des galactomannanes cationiques dont les propriétés en termes de dépôt de silicone sont comparables à celles de guars cationiques connus.

## Revendications

1. Procédé de préparation de galactomannane cationique, comprenant les étapes suivantes :

   a) une étape d'imprégnation de galactomannane sous forme de splits par un agent alcalin ;
   b) une étape d'imprégnation par un agent cationique du mélange formé à l'issue de l'étape a) ; et
   c) une étape de séchage du mélange formé à l'issue de l'étape b).

2. Procédé selon la revendication 1, dans lequel le galactomannane est choisi parmi les guars.

3. Procédé selon l'une quelconque des revendications 1 à 2, **caractérisé en ce qu'**il est effectué en continu.

4. Procédé selon l'une quelconque des revendications 1 à 3, dans laquelle l'étape a) est effectuée pendant une durée d'imprégnation d'au moins 15 minutes, par exemple comprise entre 15 minutes et 40 minutes, par exemple d'au moins 20 minutes, par exemple comprise entre 20 et 30 minutes, à une température comprise entre 55°C et 65°C.

5. Procédé selon l'une quelconque des revendications 1 à 4, dans laquelle l'étape b) est effectuée pendant une durée d'imprégnation d'au moins 15 minutes, par exemple comprise entre 15 minutes et 40 minutes, par exemple d'au moins 20 minutes, par exemple comprise entre 20 et 30 minutes, à une température comprise entre 55°C et 65°C.

6. Procédé selon l'une quelconque des revendications 1 à 5, dans lequel l'étape a) comprend une étape d'addition d'une solution aqueuse de l'agent alcalin sur les galactomannanes maintenus à une température inférieure à 90°C, notamment inférieure à 80°C, de préférence inférieure à 70°C, et préférentiellement inférieure à 65°C, ou encore en particulier compris e de 55°C à 65°C.

7. Procédé selon l'une quelconque des revendications 1 à 6, dans lequel la solution aqueuse de l'agent alcalin est ajoutée à une température inférieure à 90°C, notamment inférieure à 80°C, de préférence inférieure à 70°C, et préférentiellement inférieure à 65°C, ou encore en particulier compris e de 55°C à 65°C, et de préférence égale à environ 60°C.

8. Procédé selon l'une quelconque des revendications 1 à 7, dans lequel l'agent alcalin est utilisé en excès par rapport à l'agent cationique.

9. Procédé selon la revendication 8, dans lequel le rapport entre le nombre de moles de l'agent alcalin et le nombre de moles de l'agent cationique est compris de 1,5 à 2,5, et de préférence égale à 1,7.

10. Procédé selon l'une quelconque des revendications 1 à 9, dans lequel, lors de l'étape c), la température de l'air de séchage est supérieure ou égale à 60°C, notamment comprise entre 60°C et 150°C, de préférence entre 60°C et 100°C, et de préférence égale à environ 80°C.

11. Procédé selon l'une quelconque des revendications 1 à 10, dans lequel la durée de séchage est supérieure ou égale à 5 minutes, par exemple supérieure ou égale à 10 minutes, par exemple comprise entre 10 minutes et 180 minutes, et est notamment supérieure ou égale à 15 minutes.

12. Procédé selon l'une quelconque des revendications 1 à 11, dans lequel le galactomannane cationique obtenu à l'issue de l'étape de séchage présente un taux d'humidité inférieur à 5%.

13. Procédé selon l'une quelconque des revendications 1 à 12, dans lequel l'agent alcalin est la soude.

14. Procédé selon l'une quelconque des revendications 1 à 13, **caractérisé en ce qu'**il ne comprend pas d'étape de lavage.

## Patentansprüche

1. Verfahren zur Herstellung von kationischem Galactomannan, umfassend die folgenden Schritte:

   a) einen Schritt der Imprägnierung von Galactomannan in Form von Splits mit einem alkalischen Mittel,

b) einen Schritt der Imprägnierung des am Ende von Schritt a) gebildeten Gemischs mit einem kationischen Mittel und
c) einen Schritt des Trocknens des am Ende von Schritt b) gebildeten Gemischs.

**2.** Verfahren nach Anspruch 1, wobei das Galactomannan aus Guaren ausgewählt ist.

**3.** Verfahren nach einem der Ansprüche 1 bis 2, **dadurch gekennzeichnet, dass** es im Durchlaufbetrieb durchgeführt wird.

**4.** Verfahren nach einem der Ansprüche 1 bis 3, wobei Schritt a) während einer Imprägnierungsdauer von mindestens 15 Minuten, zum Beispiel zwischen 15 Minuten und 40 Minuten, zum Beispiel von mindestens 20 Minuten, zum Beispiel zwischen 20 und 30 Minuten, bei einer Temperatur zwischen 55 °C und 65 °C durchgeführt wird.

**5.** Verfahren nach einem der Ansprüche 1 bis 4, wobei Schritt b) während einer Imprägnierungsdauer von mindestens 15 Minuten, zum Beispiel zwischen 15 Minuten und 40 Minuten, zum Beispiel von mindestens 20 Minuten, zum Beispiel zwischen 20 und 30 Minuten, bei einer Temperatur zwischen 55 °C und 65 °C durchgeführt wird.

**6.** Verfahren nach einem der Ansprüche 1 bis 5, wobei Schritt a) einen Schritt der Zugabe einer wässrigen Lösung des alkalischen Mittels zu den Galactomannanen umfasst, die bei einer Temperatur unter 90 °C, insbesondere unter 80 °C, vorzugsweise unter 70 °C und bevorzugt unter 65 °C oder auch insbesondere zwischen 55 °C bis 65°C gehalten werden.

**7.** Verfahren nach einem der Ansprüche 1 bis 6, wobei eine wässrige Lösung des alkalischen Mittels bei einer Temperatur unter 90 °C, insbesondere unter 80 °C, vorzugsweise unter 70 °C und bevorzugt unter 65 °C oder auch insbesondere zwischen 55 °C bis 65°C und vorzugsweise gleich etwa 60 °C zugegeben wird.

**8.** Verfahren nach einem der Ansprüche 1 bis 7, wobei das alkalische Mittel im Überschuss im Verhältnis zum kationischen Mittel verwendet wird.

**9.** Verfahren nach Anspruch 8, wobei das Verhältnis zwischen der Anzahl Mole des alkalischen Mittels und der Anzahl Mole des kationischen Mittels zwischen 1,5 bis 2,5 und vorzugsweise gleich 1,7 beträgt.

**10.** Verfahren nach einem der Ansprüche 1 bis 9, wobei während Schritt c) die Temperatur der Trocknungsluft höher als oder gleich 60 °C, insbesondere zwischen 60 °C und 150 °C, vorzugsweise zwischen 60 °C und 100 °C und bevorzugt gleich etwa 80 °C ist.

**11.** Verfahren nach einem der Ansprüche 1 bis 10, wobei die Trocknungsdauer länger als oder gleich 5 Minuten, zum Beispiel länger als oder gleich 10 Minuten, zum Beispiel zwischen 10 Minuten und 180 Minuten ist und insbesondere länger als oder gleich 15 Minuten ist.

**12.** Verfahren nach einem der Ansprüche 1 bis 11, wobei das am Ende des Trocknungsschritts erhaltene kationische Galactomannan einen Feuchtigkeitsgehalt unter 5% aufweist.

**13.** Verfahren nach einem der Ansprüche 1 bis 12, wobei das alkalische Mittel Natriumhydroxid ist.

**14.** Verfahren nach einem der Ansprüche 1 bis 13, **dadurch gekennzeichnet, dass** es keinen Waschschritt umfasst.

## Claims

**1.** Process for preparing cationic galactomannan, comprising the following steps:

a) a step of impregnating galactomannan in the form of splits with an alkaline agent;
b) a step of impregnating the mixture formed on conclusion of step a) with a cationic agent; and
c) a step of drying the mixture formed on conclusion of step b) .

**2.** Process according to Claim 1, in which the galactomannan is chosen from guars.

3. Process according to any one of Claims 1 and 2, **characterized in that** it is performed continuously.

4. Process according to any one of Claims 1 to 3, in which step a) is performed for an impregnation time of at least 15 minutes, for example between 15 minutes and 40 minutes, for example of at least 20 minutes, for example between 20 minutes and 30 minutes, at a temperature of between 55°C and 65°C.

5. Process according to any one of Claims 1 to 4, in which step b) is performed for an impregnation time of at least 15 minutes, for example between 15 minutes and 40 minutes, for example of at least 20 minutes, for example between 20 minutes and 30 minutes, at a temperature of between 55°C and 65°C.

6. Process according to any one of Claims 1 to 5, in which step a) comprises a step of addition of an aqueous solution of the alkaline agent to the galactomannans maintained at a temperature below 90°C, notably below 80°C, preferably below 70°C and preferentially below 65°C, or alternatively in particular from 55°C to 65°C.

7. Process according to any one of Claims 1 to 6, in which the aqueous solution of the alkaline agent is added at a temperature below 90°C, notably below 80°C, preferably below 70°C and preferentially below 65°C, or alternatively in particular from 55°C to 65°C, and preferably equal to about 60°C.

8. Process according to any one of Claims 1 to 7, in which the alkaline agent is used in excess relative to the cationic agent.

9. Process according to Claim 8, in which the ratio between the number of moles of the alkaline agent and the number of moles of the cationic agent is from 1.5 to 2.5 and is preferably equal to 1.7.

10. Process according to any one of Claims 1 to 9, in which, during step c), the drying air temperature is greater than or equal to 60°C, notably between 60°C and 150°C, preferably between 60°C and 100°C, and preferably equal to about 80°C.

11. Process according to any one of Claims 1 to 10, in which the drying time is greater than or equal to 5 minutes, for example greater than or equal to 10 minutes, for example between 10 minutes and 180 minutes, and is notably greater than or equal to 15 minutes.

12. Process according to any one of Claims 1 to 11, in which the cationic galactomannan obtained on conclusion of the drying step has a moisture content of less than 5%.

13. Process according to any one of Claims 1 to 12, in which the alkaline agent is sodium hydroxide.

14. Process according to any one of Claims 1 to 13, **characterized in that** it does not comprise a washing step.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 4940784 A
- US 2009197829 A